# EUROPÄISCHE PATENTSCHRIFT

(11) **EP 2 864 284 B1**
(45) Veröffentlichungstag und Bekanntmachung des Hinweises auf die Patenterteilung: **05.07.2017**
(21) Anmeldenummer: 13721305.4
(22) Anmeldetag: 26.04.2013
(51) Int. Cl.: C07C 57/38, C07C 69/738, C11B 9/00, A61K 8/365, A61K 8/37, A61Q 13/00, C11D 3/50

(54) **PHOTOLABILE DUFTSPEICHERSTOFFE**
PHOTOLABILE PRO-FRAGRANCES
SUBSTANCES PHOTOLABILES RENFERMANT UN PARFUM

(30) Priorität: 22.06.2012 DE 102012210567; 05.09.2012 DE 102012215693
(43) Veröffentlichungstag der Anmeldung: 29.04.2015
(73) Patentinhaber: Henkel AG & Co. KGaA, 40589 Düsseldorf (DE)
(72) Erfinder: GERKE, Thomas, 40627 Düsseldorf (DE); KROPF, Christian, 40724 Hilden (DE); HUCHEL, Ursula, 50733 Köln (DE); GRIESBECK, Axel, 50937 Köln (DE); LANDES, Agnieszka, 50129 Bergheim (DE)
(86) Internationale Anmeldenummer: PCT/EP2013/058681
(87) Internationale Veröffentlichungsnummer: WO 2013/189646

(56) Entgegenhaltungen:
- WO-A1-2004/045616
- WO-A1-2011/101180
- WO-A2-2004/099132
- BASU B ET AL: "KF-alumina mediated selective double Michael additions of aryl methyl ketones: A facile entry to the synthesis of functionalized pimelate esters and derivatives", SYNLETT, GEORG THIEME VERLAG, DE, Bd. 2004, Nr. 12, 1. Januar 2004 (2004-01-01), Seiten 2224-2226, XP002649372, ISSN: 0936-5214, DOI: 10.1055/S-2004-831339
- JOHANN MULZER ET AL: "Additionen von Carbonsäure-Dianionen an [alpha], [beta]-ungesättigte Carbonylverbindungen - Steuerung der 1,2-/1,4-Regioselektivität durch sterische Substituenteneffekte", CHEMISCHE BERICHTE, Bd. 114, Nr. 11, 1. November 1981 (1981-11-01), Seiten 3701-3724, XP055064770, ISSN: 0009-2940, DOI: 10.1002/cber.19811141123
- E. P. KOHLER ET AL: JOURNAL OF THE AMERICAN CHEMICAL SOCIETY, Bd. 41, Nr. 6, 1. Juni 1919 (1919-06-01), Seiten 992-1001, XP055064771, ISSN: 0002-7863, DOI: 10.1021/ja02227a009
- YOSHIHARU ONISHI ET AL: "InCl 3 /Me 3 SiBr-Catalyzed Direct Coupling between Silyl Ethers and Enol Acetates", ORGANIC LETTERS, Bd. 13, Nr. 10, 20. Mai 2011 (2011-05-20), Seiten 2762-2765, XP055064872, ISSN: 1523-7060, DOI: 10.1021/ol200875m
- R. SHINTANI; G.C. FU: "Highly Enantioselective Desymmetrization of Anhydrides by Carbon Nucleophiles: Reactions of Grignard Reagents in the Presence of (-)-Sparteine", ANGEW. CHEM. INT. ED., Bd. 41, Nr. 6, 2002, Seiten 1057-1059, XP002698014, ISSN: 1433-7851
- B.B. GHATGE ET AL.: "Preparation of beta-aryl Butyric Acids: the Attempted Synthesis of Hydantoin Analogues", J. INDIAN. CHEM. SOC., Bd. 58, 1981, Seiten 90-91, XP008162603, ISSN: 0019-4522
- W.S. MURPHY; S. WATTANASIN: "Reductive Cleavage of Arylcyclopropyl Ketones", J. CHEM. SOC. PERKIN TRANS. I, 1986, Seiten 1445-1451, XP002698015, ISSN: 0300-922X
- WON SUK SHIN ET AL.: "Effect of the alkyl chain length of C70-PCBX acceptors on the device performance of P3HT: C70-PCBX polymer solar cells", J. MATER. CHEM., Bd. 21, 2011, Seiten 960-967, XP002698066, ISSN: 0959-9428

## Beschreibung

Die vorliegende Erfindung betrifft spezielle Verbindungen, die als photolabile Duftspeicherstoffe für Duftstoffsäuren und -ester fungieren. Ferner betrifft die vorliegende Erfindung Wasch- oder Reinigungsmittel, kosmetische Mittel sowie Raumbeduftungsmittel, welche solche Verbindungen enthalten. Ferner betrifft sie ein Verfahren zur lang anhaltenden Beduftung von Oberflächen und ebenso ein Verfahren zur lang anhaltenden Raumbeduftung.

Wasch- oder Reinigungsmittel bzw. kosmetische Mittel enthalten zumeist Duftstoffe, die den Mitteln einen angenehmen Geruch verleihen. Die Duftstoffe maskieren dabei auch einen eventuellen Geruch der anderen Inhaltstoffe, so dass beim Verbraucher ein angenehmer Geruchseindruck entsteht.

Insbesondere im Bereich Waschmittel sind Duftstoffe wichtige Bestandteile der Zusammensetzung, da die Wäsche sowohl im feuchten als auch im trockenen Zustand einen angenehmen und möglichst auch frischen Duft aufweisen soll. Man steht beim Einsatz von Duftstoffen grundsätzlich vor dem Problem, dass es sich bei diesen um mehr oder minder leicht flüchtige Verbindungen handelt, aber dennoch ein lange anhaltender Dufteffekt angestrebt wird. Insbesondere bei denjenigen Riechstoffen, die die frischen und leichten Noten des Parfüms darstellen und infolge ihres hohen Dampfdruck besonders schnell abdampfen, ist die gewünschte Langlebigkeit des Dufteindrucks kaum erreichbar.

Eine verzögerte Duftfreisetzung kann z.B. durch Träger-gebundenen Einsatz von Duftstoffen erfolgen. Eine Träger-gebundene Vorform eines Duftstoffes wird auch als "Pro-Fragrance" oder Duftspeicherstoff bezeichnet. In diesem Zusammenhang offenbart die internationale Patentanmeldung WO2007/087977 die Verwendung von 1-Aza-3,7-dioxabicyclo[3.3.0]octan-Verbindungen als Duftspeicherstoffe zur verzögerten Freisetzung von Duftaldehyden und Duftketonen durch Hydrolyse. Eine alternative Möglichkeit der verzögerten Freisetzung von Duftstoffen stellt der Einsatz von so genannten photoaktivierbaren Substanzen als Duftspeicherstoffe dar. Durch die Einwirkung des Sonnenlichts oder einer anderen elektromagnetischen Strahlungsquelle bestimmter Wellenlänge wird der Bruch einer kovalenten Bindung im Duftspeicherstoff-Molekül induziert, wodurch ein Duftstoff freigesetzt wird.

Die WO 2011/101180 offenbart die Verwendung bestimmter Ketone als photoaktivierbare Substanzen, die in Gegenwart von Licht in einer photochemischen Fragmentierung einen Aktivstoff freisetzen. Der genannte Aktivstoff besitzt beispielsweise eine duftgebende Aktivität, die durch den photochemisch induzierten Zerfall erst verzögert und über einen längeren Zeitraum hinweg auf einer bestimmten Oberfläche freigesetzt wird. Allerdings ist bislang lediglich die Freisetzung von Duftstoffketonen bekannt. Die verzögerte Freisetzung anderer Duftstoffe als Ketone ist jedoch wichtig, um ein möglichst breites Spektrum an lang anhaltenden Dufteindrücken für den Verbraucher bereitstellen zu können.

Aufgabe der vorliegenden Erfindung war die Bereitstellung photoaktivierbarer Substanzen als Duftspeicherstoffe, welche die verzögerte Freisetzung von Duftstoffestern, insbesondere von Zimtsäureestern und Derivaten der Zimtsäureester erlauben.

Gelöst wurde diese Aufgabe durch eine Verbindung der allgemeinen Formel (I), 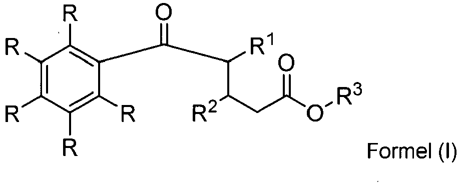 wobei
R1 für Wasserstoff, eine lineare oder verzweigte, substituierte oder unsubstituierte Alkyl-, Aryl-, oder Alkenylgruppe,
R2 und R3 unabhängig voneinander für eine lineare oder verzweigte, substituierte oder unsubstituierte Alkyl-, Aryl-, oder Alkenylgruppe, und
R jeweils, unabhängig voneinander, für Wasserstoff, eine Aminogruppe, -NO₂, eine lineare oder verzweigte, substituierte oder unsubstituierte Alkoxygruppe mit 1 bis 15 C-Atomen, eine lineare oder verzweigte, substituierte oder unsubstituierte Alkylgruppe mit 1 bis 15 C-Atomen, eine Cycloalkylgruppe, Acylgruppe, Arylgruppe, -OH, -NH₂, Halogen, NH-Alkyl oder -N(Alkyl)₂ stehen, wobei der Substituent R3 eine n-Pentyl, sec-Pentyl-, Cinnamyl- oder Citronellylgruppe ist.

Es konnte überraschend gefunden werden, dass die erfindungsgemäßen Verbindungen der allgemeinen Formel (I) besonders wirksame Duftspeicherstoffe sind, welche die verzögerte Freisetzung von Duftstoffsäuren und -estern, insbesondere von Duftstoffestern und insbesondere Zimtsäurestern und Derivaten der Zimtsäureester erlauben. Die Anwendung der erfindungsgemäßen Verbindungen in Wasch- oder Reinigungsmitteln, kosmetischen Mitteln, oder Raumbeduftungsmitteln führte bei deren Anwendung zu einer verbesserten Langzeitduftwirkung, insbesondere im Zusammenhang mit der Textilbehandlung. So konnte bei der Anwendung erfindungsgemäßer Verbindungen in einem Wäschebehandlungsmittel, wie z.B. Waschmittel sowie Weichspüler, eine verbesserte Langzeitduftwirkung der behandelten Wäsche gefunden werden. Ferner weisen entsprechende

Produkte eine besonders gute Lagerstabilität auf. Die erfindungsgemäßen Mittel ermöglichen es zudem, die Gesamtmenge an Parfüm, welche im Mittel enthalten ist, zu reduzieren, und dennoch Geruchsvorteile auf den gewaschenen Textilien zu erzielen, insbesondere mit Blick auf das Frischeempfinden.

Eine bevorzugte Ausführüngsform der vorliegenden Erfindung betrifft insbesondere Duftstoffspeicherstoffe von Duftstoffestern.

Die erfindungsgemäße Verbindung gemäß der allgemeinen Formel (I) ist als Duftspeicherstoff für alle üblichen Duftstoffester geeignet, die in ihrer freien Form als Duftstoff eine alpha,beta-ungesättigte Carbonyleinheit oder mesomere Grenzformen davon aufweisen. Bevorzugte Duftstoffester sind ausgewählt aus Zimtsäureestern und Derivaten der Zimtsäureester. Insbesondere bevorzugt sind Linaloylcinnamat, 3-Phenylpropylcinnamat, Eugenolcinnamat, Allylcinnamat, Benzylcinnamat, Butylcinnamat, Ethylcinnamat, Methylcinnamat, Menthylcinnamat, Heptylcinnamat, Cylclohexylcinnamat, Isoamylcinnamat, Isobutylcinnamat, Isopentylcinnamat, Isopropylcinnamat, Isoheptylcinnamat, Tetrahydrofurfurylcinnamat und Cinnamylcinnamat. Durch Einwirkung von Licht umfassend die Wellenlängen von 200 bis 400 nm können die gespeicherten Ester freigesetzt werden.

Gemäß einer bevorzugten Ausführungsform der Erfindung steht der Substituent R2 in der Formel (I) für eine substituierte oder unsubstituierte Arylgruppe, insbesondere für ein substituiertes oder unsubstituiertes Phenyl, besonders bevorzugt für ein unsubstituiertes Phenyl.

Gemäß einer weiteren bevorzugten Ausführungsform der Erfindung steht der Substituent R1 in der Formel (I) für eine lineare oder verzweigte, substituierte oder unsubstituierte Alkylgruppe mit 1 bis 6 C-Atomen, vorzugsweise 1 bis 3 C-Atomen, insbesondere handelt es sich um einen Methylrest.

In der Formel (I) steht der Substituent R3 für eine n-Pentyl, sec-Pentyl-, Cinnamyl- oder Citronellylgruppe.

Gemäß einer weiteren bevorzugten Ausführungsform der Erfindung ist eine erfindungsgemäße Verbindung der allgemeinen Formel (I) bevorzugt, in der alle Substituenten R für Wasserstoff stehen. In einer weiteren bevorzugten Ausführungsform der Erfindung ist eine erfindungsgemäße Verbindung der allgemeinen Formel (I) bevorzugt, in der vier der fünf Arylsubstituenten R für Wasserstoff stehen. Vorzugsweise stehen die vier Reste R in ortho- und meta-Position des Aromaten für Wasserstoff, während der Rest R in para-Position des Aromaten für ein Halogenatom, insbesondere -F, -Cl oder -Br, -NO₂, eine lineare oder verzweigte, substituierte oder unsubstituierte Alkoxygruppe mit 1 bis 6 C-Atomen oder eine lineare oder verzweigte, substituierte oder unsubstituierte Alkylgruppe mit 1 bis 6 C-Atomen steht. In einer weiter bevorzugten Ausführungsform der Erfindung steht der Rest R in para-Position des Aromaten in der Formel (I) für -Cl, -Br, -NO₂ oder eine Alkyl- oder Alkoxygruppe umfassend 1 bis 4 C-Atome. Vorzugsweise handelt es sich bei dem Rest R in para-Position des Aromaten um eine Methyl- oder Ethylgruppe oder um eine Methoxy-, Ethoxy-, Isopropoxy- oder tert-Butoxygruppe. Eine Substitution in para-Position ist besonders bevorzugt, da die elektronische Struktur des aromatischen Rings hier am effektivsten modifiziert werden kann, wodurch das Absorptionsmaximum von Verbindungen der allgemeinen Formel (I) leicht an eine bestimmte Wellenlänge angepasst werden kann.

Ganz besonders bevorzugt ist es, wenn in der Formel (I) einer der beiden Reste R in meta-Position des Aromaten für eine Alkoxygruppe, insbesondere eine Methoxygruppe, und der Rest R in para-Position des Aromaten für eine Alkoxygruppe, insbesondere eine Methoxygruppe steht und der andere Rest R in meta-Position des Aromaten so wie die beiden Reste R in ortho-Position des Aromaten für Wasserstoff stehen.

Gemäß einer bevorzugten Ausführungsform der Erfindung entsprechen Verbindungen der Formel (I) den nachfolgenden Formeln (XX)-(ILIV):

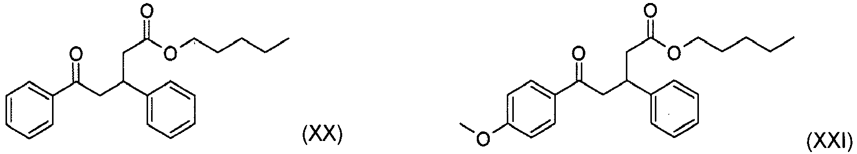

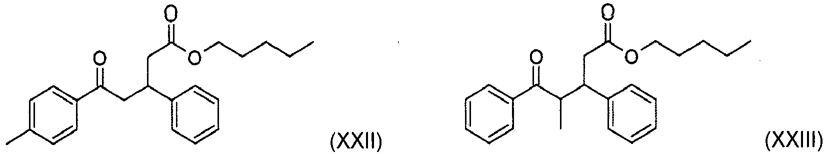

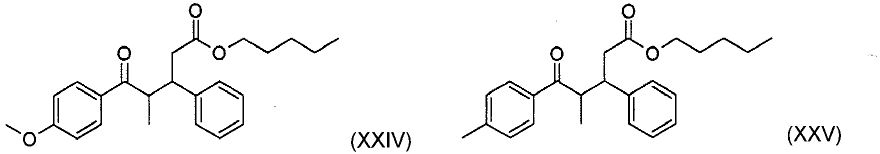

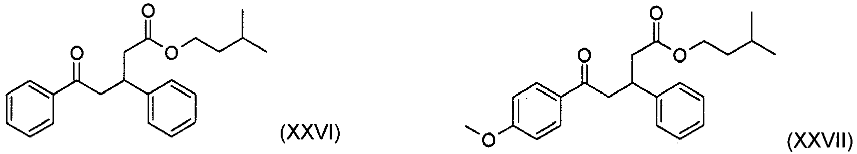

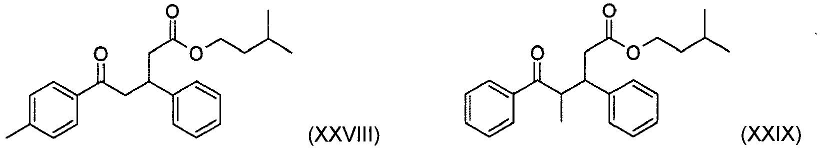

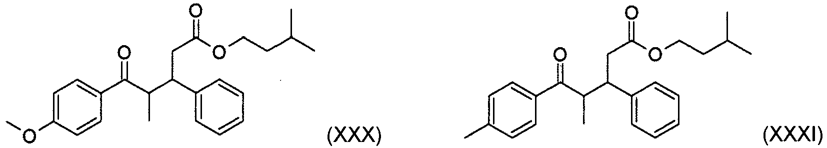

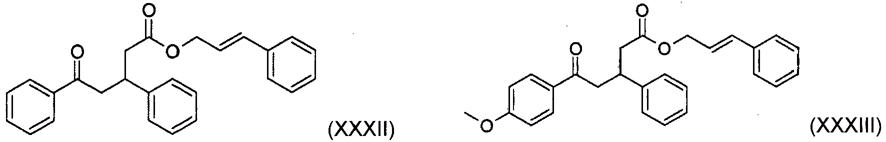

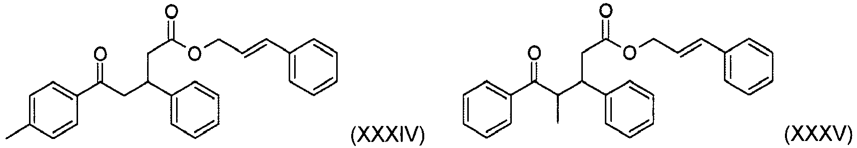

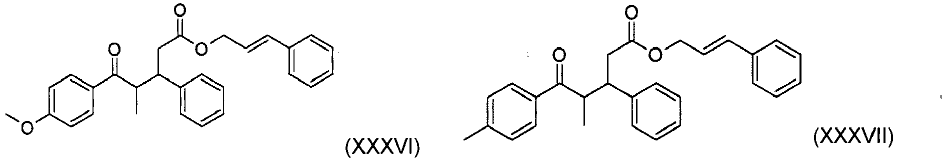

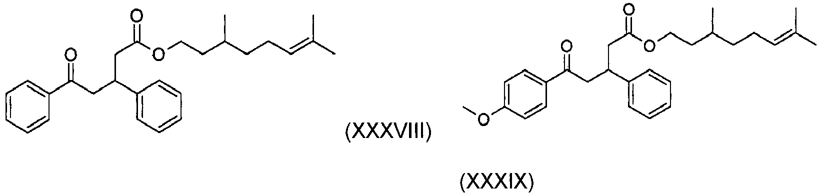

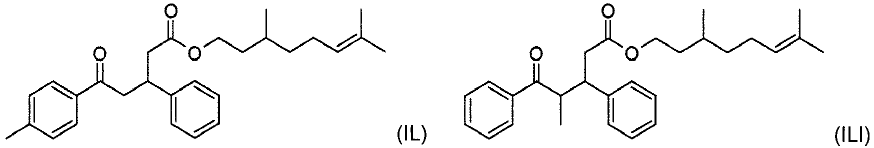

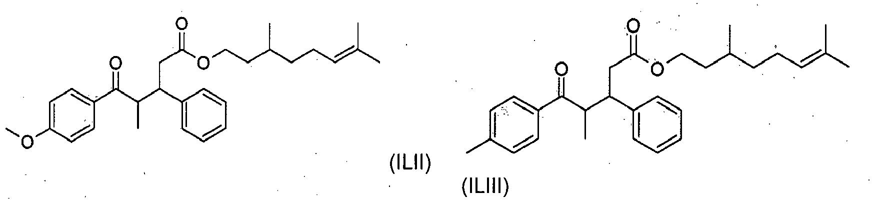

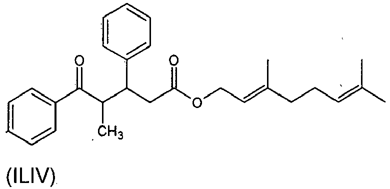

Verbindungen gemäß Formel (I), insbesondere die Verbindungen der vorgenannten Formeln (II) bis (ILIV), lassen sich stabil in die üblichen Wasch- oder Reinigungsmittelmatrices, in Kosmetika und bestehende Riechstoffkompositionen einarbeiten. Sie ermöglichen eine verzögerte Freisetzung der gespeicherten Duftstoffe, nämlich von Zimtsäurestern oder von Derivaten der Zimtsäureester. Diese Verbindungen verleihen üblichen Wasch- oder Reinigungsmitteln sowie Kosmetika einen besonders lange anhaltenden Frischeeindruck. Insbesondere das getrocknete, gewaschene Textil profitiert von der guten Langzeitfrischeduftwirkung. Die langsame Freisetzung des gespeicherten Riechstoffes erfolgt nach Einwirkung von Licht (elektromagnetische Strahlung) umfassend die Wellenlängen von 200 bis 400 nm, wie in der nachfolgender Reaktionsgleichung vereinfacht veranschaulicht:

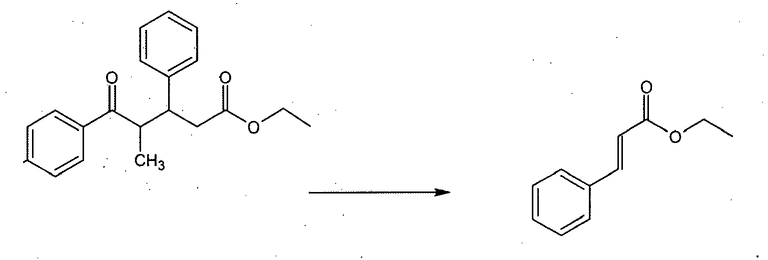

Die Verwendung von Verbindungen der Formel (I), die durch Einführung spezieller Substituenten R, bevorzugt Methyl oder Methoxy, modifiziert wurden, führt zu einer Lichtabsorption in einem höheren Wellenlängenbereich. Bei Exposition dieser Verbindungen in natürlichem Licht oder Licht, das durch haushaltsübliche Leuchtmittel erzeugt wird, werden besonders rasch über dem Geruchschwellenwert liegende Riechstoffmengen freigesetzt, wodurch die Effektivität der Verbindungen der Formel (I) erhöht wird.

Ein weiterer Gegenstand der vorliegenden Erfindung ist ein Wasch- oder Reinigungsmittel, vorzugsweise ein Waschmittel, Weichspüler oder Waschhilfsmittel, enthaltend mindestens eine Verbindung gemäß einer der Formeln (I) bis (ILIV), wobei die genannte Verbindung vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,05 und 2 Gew.-%, jeweils bezogen auf das gesamte Mittel, enthalten ist. Geeignete Reinigungsmittel sind z.B. Reinigungsmittel für harte Oberflächen, wie vorzugsweise Geschirrspülmittel. Ebenso kann es sich um Reinigungsmittel wie z.B. Haushaltsreiniger, Allzweckreiniger, Fensterreiniger, Fußbodenreiniger usw. handeln. Vorzugsweise kann es sich um ein Produkt zur Reinigung von WC-Becken und Urinalen handeln, vorteilhafterweise um einen Spülreiniger zum Einhängen in das WC-Becken, insbesondere um einen sogenannten WC-Stein.

Gemäß einer bevorzugten Ausführungsform der Erfindung enthält das erfindungsgemäße Wasch- oder Reinigungsmittel mindestens ein Tensid, ausgewählt aus anionischen, kationischen, nichtionischen, zwitterionischen, amphoteren Tensiden oder Mischungen daraus.

Gemäß einer weiteren bevorzugten Ausführungsform der Erfindung liegt das erfindungsgemäße Mittel in fester oder flüssiger Form vor.

Ein weiterer Gegenstand der Erfindung ist ein kosmetisches Mittel, enthaltend mindestens eine Verbindung gemäß einer der Formeln (I) bis (ILIV), welches die genannte Verbindung vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,05 und 2 Gew.-%, jeweils bezogen auf das gesamte Mittel, enthält.

Ein weiterer Gegenstand der Erfindung ist ein Raumbeduftungsmittel (z.B. Raumlufterfrischer, Raumdeodorant, Raumspray usw.), enthaltend mindestens eine Verbindung gemäß einer der Formeln (I) bis (ILIV), wobei die genannte Verbindung vorzugsweise in Mengen zwischen 0,0001 und 50 Gew.-%, vorteilhafterweise zwischen 0,001 und 5 Gew.-%, weiter vorteilhaft zwischen 0,1 und 3 Gew.-%, insbesondere zwischen 0,1 und 2 Gew.-%, jeweils bezogen auf das gesamte Mittel, enthalten ist.

Nach einer weiteren bevorzugten Ausführungsform der Erfindung sind in einem erfindungsgemäßen Mittel (d.h. Wasch- oder Reinigungsmittel, kosmetisches Mittel, oder Raumbeduftungsmittel) zusätzliche Duftstoffe enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Haftfeste Duftstoffe, die im Rahmen der vorliegenden Erfindung mit Vorteil einsetzbar sind, sind beispielsweise etherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch höhersiedende bzw. feste Duftstoffe natürlichen oder synthetischen Ursprungs können im Rahmen der vorliegenden Erfindung als haftfeste Duftstoffe bzw. Duftstoffgemische, also Duftstoffe eingesetzt werden. Zu diesen Verbindungen zählen die nachfolgend genannten Verbindungen sowie Mischungen aus diesen: Ambrettolid, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon- Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-beta-naphthylketon, Methyl-n-nonylacetaldehyd, Methyl-n-nonylketon, Muskon, beta-Naphtholethylether, beta-Naphtholmethylether, Nerol, Nitrobenzol, n-Nonylaldehyd, Nonylakohol, n-Octylaldehyd, p-Oxy-Acetophenon, Pentadekanolid, beta-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, gamma-Undelacton, Vanillin, Veratrumaldehyd, Zimtaldehyd, Zimtalkohol, Zimtsäure, Zimtsäureethylester, Zimtsäurebenzylester.

Zu den leichter flüchtigen Duftstoffen zählen insbesondere die niedriger siedenden Duftstoffe natürlichen oder synthetischen Ursprungs, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Nach einer weiteren bevorzugten Ausführungsform weist das erfindungsgemäße Mittel, d.h. Wasch- oder Reinigungsmittel, kosmetische Mittel, oder Raumbeduftungsmittel, wenigstens eine, vorzugsweise mehrere, aktive Komponenten, insbesondere wasch-, pflege-, reinigungsaktive und/oder kosmetische Komponenten auf, vorteilhafterweise ausgewählt aus der Gruppe umfassend anionische Tenside, kationische Tenside, amphotere Tenside, nichtionische Tenside, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Duftstoffe, Einlaufverhinderer, Elektrolyte, Enzyme, Farbschutzstoffe, Färbemittel, Farbstoffe, Farbübertragungsinhibitoren, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, Soilrelease-Wirkstoffe, UV-Schutz-Substanzen, Viskositätsregulatoren, Verdickungsmittel, Vitamine und/oder weichmachende Verbindungen. Im Sinne dieser Erfindung beziehen sich Angaben für das erfindungsgemäße Mittel in Gew.-%, wenn nicht anders angegeben, auf das Gesamtgewicht des erfindungsgemäßen Mittels.

Die Mengen der einzelnen Inhaltsstoffe in den erfindungsgemäßen Mitteln, d.h. Wasch- oder Reinigungsmittel, kosmetisches Mittel oder Raumbeduftungsmittel, orientieren sich jeweils am Einsatzzweck der betreffenden Mittel und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der erfindungsgemäßen Mittel wird man beispielsweise den Tensidgehalt höher oder niedriger wählen.

Die erfindungsgemäßen Mittel (d.h. Wasch- oder Reinigungsmittel, kosmetisches Mittel oder Raumbeduftungsmittel) können Tenside enthalten, wobei bevorzugt anionische Tenside, nichtionische Tenside und deren Gemische, aber auch kationische Tenside in Frage kommen.

Tenside sind gegebenenfalls in den erfindungsgemäßen Mitteln, d.h. Wasch- oder Reinigungsmittel, kosmetisches Mittel, oder Raumbeduftungsmittel, in Mengenanteilen von vorzugsweise 5 Gew.-% bis 50 Gew.-%, insbesondere von 8 Gew.-% bis 30 Gew.-%, enthalten. Insbesondere in Wäschenachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder Sulfonatgruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X- für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer MethylGruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden z.B. mit Dimethylsulfat quaterniert. In Frage kommende QAV sind beispielweise Benzalkoniumchlorid (N Alkyl-N,N-dimethyl-benzylammoniumchlorid), Benzalkon B (m,p-Dichlor-benzyldimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N-Dimethyl-N [2-[2-[p-(1,1,3,3-tetramethylbutyl)phenoxy]-ethoxy]-ethyl]-benzylammoniumchlorid), Dialkyldimethylammoniumchloride wie Di-n-decyl-dimethyl-ammoniumchlorid, Didecyldimethylammoniumbromid, Dioctyldimethylammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkyl-benzyl-dimethylammoniumchlorid.

Bevorzugte Esterquats sind Methyl-N-(2-hydroxyethyl)-N,N-di(talgacyl-oxyethyl)ammonium-metho-sulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Handelsübliche Beispiele sind die von der Firma Stepan unter dem Warenzeichen Stepantex^{®} vertriebenen Methylhydroxyalkyldialkoyloxyalkylammoniummethosulfate oder die unter dem Handelsnamen Dehyquart^{®} bekannten Produkte der Firma BASF SE beziehungsweise die unter der Bezeichnung Rewoquat^{®} bekannten Produkte des Herstellers Evonik Industries AG.

Ein erfindungsgemäßes Mittel, insbesondere Wasch- oder Reinigungsmittel, enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Alle genannten Säuren werden in der Regel in Form ihrer wasserlöslichen Salze, insbesondere ihre Alkalisalze, eingesetzt.

Organische Buildersubstanzen können gewünschtenfalls in Mengen bis zu 40 Gew.-%, insbesondere bis zu 25 Gew.-% und vorzugsweise von 1 bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Erfindungsgemäße Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht, beispielsweise Zeolith A, P oder X.

Anorganische Buildersubstanzen sind gewünschtenfalls in den erfindungsgemäßen Mitteln vorzugsweise in Mengen bis zu 60 Gew.-%, insbesondere von 5 bis 40 Gew.-%, enthalten. Erfindungsgemäße Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Der Zusatz geringer Mengen bekannter Bleichmittelstabilisatoren wie beispielsweise von Phosphonaten, Boraten bzw. Metaboraten und Metasilikaten sowie Magnesiumsalzen wie Magnesiumsulfat kann zweckdienlich sein.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind gewünschtenfalls in den erfindungsgemäßen Mitteln vorzugsweise in Mengen nicht über 5 Gew.-%, insbesondere von 0,2 bis 2 Gew.-%, enthalten.

Die Herstellung der erfindungsgemäßen Verbindungen wird im Beispielteil exemplarisch anhand der Herstellung eines Duftspeicherstoffes, der einen Zimtsäureester enthält, beschrieben. Über diese prinzipielle Syntheseroute sind auch die anderen Verbindungen der allgemeinen Formel (I) und insbesondere alle Verbindungen der Formeln (II) bis (ILIV) zugänglich.

Gemäß einer bevorzugten Ausführungsform kann die erfindungsgemäße Lehre dazu eingesetzt werden, den Parfümanteil in Wasch- oder Reinigungsmitteln und kosmetischen Mitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder gar nicht verwenden können.

Ein bevorzugtes erfindungsgemäßes festes, insbesondere pulverförmiges Waschmittel kann neben der erfindungsgemäßen Verbindung insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5 bis 30 Gew.-%
- Nichtionische Tenside, wie vorzugsweise Fettalkoholpolyglycolether, Alkylpolyglucosid, Fettsäureglucamid z.B. in Mengen von vorzugsweise 0,5 bis 15 Gew.-%
- Gerüststoffe, wie z.B. Zeolith, Polycarboxylat, Natriumcitrat, in Mengen von z.B. 0 bis 70 Gew.-%, vorteilhafterweise 5 bis 60 Gew.-%, vorzugsweise 10 bis 55 Gew.-%, insbesondere 15 bis 40 Gew.-%,
- Alkalien, wie z.B. Natriumcarbonat, in Mengen von z.B. 0 bis 35 Gew.-% vorteilhafterweise 1 bis 30 Gew.-%, vorzugsweise 2 bis 25 Gew.-%, insbesondere 5 bis 20 Gew.-%,
- Bleichmittel, wie z.B. Natriumperborat, Natriumpercarbonat, in Mengen von z.B. 0 bis 30 Gew.-% vorteilhafterweise 5 bis 25 Gew.-%, vorzugsweise 10 bis 20 Gew.-%,
- Korrosionsinhibitoren, z.B. Natriumsilicat, in Mengen von z.B. 0 bis 10 Gew.-%, vorteilhafterweise 1 bis 6 Gew.-%, vorzugsweise 2 bis 5 Gew.-%, insbesondere 3 bis 4 Gew.-%,
- Stabilisatoren, z.B. Phosphonate, vorteilhafterweise 0 bis 1 Gew.-%,
- Schauminhibitor, z.B. Seife, Siliconöle, Paraffine vorteilhafterweise 0 bis 4 Gew.-%, vorzugsweise 0,1 bis 3 Gew.-%, insbesondere 0,2 bis 1 Gew.-%,
- Enzyme, z.B. Proteasen, Amylasen, Cellulasen, Lipasen, vorteilhafterweise 0 bis 2 Gew.-%, vorzugsweise 0,2 bis 1 Gew.-%, insbesondere 0,3 bis 0,8 Gew.-%,
- Vergrauungsinhibitor, z.B. Carboxymethylcellulose, vorteilhafterweise 0 bis 1 Gew.-%,
- Verfärbungsinhibitor, z.B. Polyvinylpyrrolidon-Derivate, vorzugsweise 0 bis 2 Gew.-%,
- Stellmittel, z.B. Natriumsulfat, vorteilhafterweise 0 bis 20 Gew.-%,
- Optische Aufheller, z.B. Stilben-Derivate, Biphenyl-Derivate, vorteilhafterweise 0 bis 0,4 Gew.-%, insbesondere 0,1 bis 0,3 Gew.-%,
- ggf. weitere Duftstoffe,
- ggf. Wasser,
- ggf. Seife,
- ggf. Bleichaktivatoren,
- ggf. Cellulosederivate,
- ggf. Schmutzabweiser,
Gew.-% jeweils bezogen auf das gesamte Mittel.

In einer anderen bevorzugten Ausführungsform der Erfindung liegt das Mittel in flüssiger Form vor, vorzugsweise in Gelform. Bevorzugte flüssige Wasch- oder Reinigungsmittel enthalten Wasser als Hauptlösungsmittel und gegebenenfalls nichtwässrige Lösungsmittel.

Ein bevorzugtes erfindungsgemäßes flüssiges, insbesondere gelförmiges Waschmittel kann neben der erfindungsgemäßen Verbindung insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5 bis 40 Gew.-%
- Nichtionische Tenside, wie vorzugsweise Fettalkoholpolyglycolether, Alkylpolyglucosid, Fettsäureglucamid z.B. in Mengen von vorzugsweise 0,5 bis 25 Gew.-%
- Gerüststoffe, wie z.B. Polycarboxylat, Natriumcitrat, vorteilhafterweise 0 bis 15 Gew.-%, vorzugsweise 0,01 bis 10 Gew.-%, insbesondere 0,1 bis 5 Gew.-%,
- Schauminhibitor, z.B. Seife, Siliconöle, Paraffine, in Mengen von z.B. 0 bis 10 Gew.-%, vorteilhafterweise 0,1 bis 4 Gew.-%, vorzugsweise 0,2 bis 2 Gew.-%, insbesondere 1 bis 3 Gew.-%,
- Enzyme, z.B. Proteasen, Amylasen, Cellulasen, Lipasen, in Mengen von z.B. 0 bis 3 Gew.-%, vorteilhafterweise 0,1 bis 2 Gew.-%, vorzugsweise 0,2 bis 1 Gew.-%, insbesondere 0,3 bis 0,8 Gew.-%,
- Optische Aufheller, z.B. Stilben-Derivat, Biphenyl-Derivat, in Mengen von z.B. 0 bis 1 Gew.-%, vorteilhafterweise 0,1 bis 0,3 Gew.-%, insbesondere 0,1 bis 0,4 Gew.-%,
- ggf. weitere Duftstoffe,
- ggf. Stabilisatoren,
- Wasser,
- ggf. Seife, in Mengen von z.B. 0 bis 25 Gew.-%, vorteilhafterweise 1 bis 20 Gew.-%, vorzugsweise 2 bis 15 Gew.-%, insbesondere 5 bis 10 Gew.-%,
- ggf. Lösungsmittel (vorzugsweise Alkohole), vorteilhafterweise 0 bis 25 Gew.-%, vorzugsweise 1 bis 20 Gew.-%, insbesondere 2 bis 15 Gew.-%, Gew.-% jeweils bezogen auf das gesamte Mittel.

Ein bevorzugter erfindungsgemäßer flüssiger Weichspüler kann neben der erfindungsgemäßen Verbindung insbesondere noch Komponenten enthalten, die ausgewählt sind aus den folgenden:
- Kationische Tenside, wie insbesondere Esterquats, z.B. in Mengen von 2bis 30 Gew.-%,
- Cotenside, wie z.B. Glycerolmonostearat, Stearinsäure, Fettalkohole, Fettalkoholethoxylate, z.B. in Mengen von 0 bis 5 Gew.-%, vorzugsweise 0,1 bis 4 Gew.-%,
- Emulgatoren, wie z.B. Fettaminethoxylate, z.B. in Mengen von 0 bis 4 Gew.-%, vorzugsweise 0,1 bis 3 Gew.-%,
- ggf. weitere Duftstoffe
- Farbstoffe, vorzugsweise im ppm-Bereich
- Stabilisatoren, vorzugsweise im ppm-Bereich
- Lösemittel, wie z.B. Wasser, in Mengen von vorzugsweise 60 bis 90 Gew.-%,
- Gew.-% jeweils bezogen auf das gesamte Mittel.

Ein weiterer Gegenstand der Erfindung ist ein Verfahren zur lang anhaltenden Beduftung von Oberflächen, wobei eine Verbindung gemäß einer der Formeln (I) bis (ILIV) oder ein erfindungsgemäßes Wasch- oder Reinigungsmittel auf die zu beduftende Oberfläche (z.B. Textil, Geschirr, Fußboden) aufgebracht wird und die genannte Oberfläche anschließend einer elektromagnetischen Strahlung umfassend die Wellenlängen von 200 bis 400 nm ausgesetzt wird.

Ein weiterer Gegenstand der Erfindung ist ein Verfahren zur lang anhaltenden Raumbeduftung, wobei ein erfindungsgemäßes Raumbeduftungsmittel einer elektromagnetischen Strahlung umfassend die Wellenlängen von 200 bis 400 nm ausgesetzt wird.

### Beispiele

### Beispiel 1: Synthese von Duftspeicherstoffen gemäß Formel (I)

### Beispiel 1a: Herstellung des Methylchalkons (AB-15)

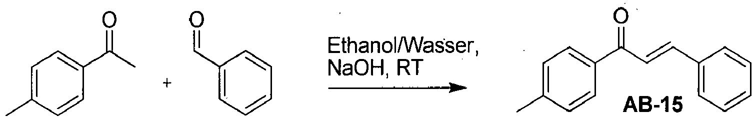

4-Methylacetophenon wurde in einem Ethanol/Wasser-Gemisch gelöst, dazu wurde NaOH gegeben und unter Eisbadkühlung langsam Benzaldehyd zugetropft. Die Reaktionsmischung wurde bei Raumtemperatur über Nacht gerührt. Das Reaktionsgemisch wurde 3 mal mit Et₂O extrahiert, mit ges. NaCl-Lösung gewaschen, danach über MgSO₄ getrocknet und unter Vakuum vom Lösemittel befreit.

Das entstandene Produkt konnte sowohl mittels ¹H-NMR- Spektroskopie sowie mit GC/MS nachgewiesen werden.
**GC/MS:** (50-300M), m/z: 222, 207, 180, 120, 92.
**t_{R}** (50-300M) = 14.276 min
**M** = 268.31 g/mol

### Beispiel 1b: Herstellung von 5-Oxo-3,5-diphenylpentansäure (AB-01)

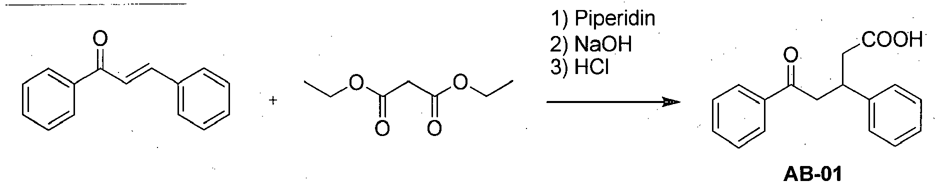

In einem 100 ml Rundkolben mit Rückflusskühler wurde eine Lösung aus Chalkon, Dimethylmalonat und Pyridin in Ethanol erhitzt und unter Rückfluss 2 Tage lang gerührt.

Nach Abkühlen der Reaktionsmischung wurde kalte 4M Natronlauge zugetropft, erhitzt und unter Rückfluss 6 Stunden lang gerührt. Anschließend wurde die Mischung abgekühlt, bei Raumtemperatur kalte konz. Salzsäure bis pH 1 zugetropft und das Reaktionsgemisch dreimal mit Diethylether extrahiert. Die vereinigten organischen Phasen wurden mit ges. NaCl-Lösüng gewaschen, danach über MgSO₄ getrocknet und unter Vakuum vom Lösemittel befreit.
**M** = 268.31 g/mol
**Smp:** 154-156 °C
**¹H-NMR:** (300 MHz, C₂D₃N)

δ (ppm) = 8.03-7.07 (m, 10H); 3.81-3.70 (m, 1H); 3.41 (d, *J* = 7.1 Hz, 2H); 2.80 (dd, *J* = 15.9, 6.4 Hz, 1 H); 2.65 (dd, *J* = 15.9, 8.6Hz, 1 H).
**¹³C-NMR:** (75 MHz, C₂D₃N):
δ (ppm) = 197.46 (C=O); 173.24 (C=O); 144.73 (Cq); 137.98 (Cq); 134.02-127.52 (C_{Ar}); 57.29 (CH); 45.11 (CH₂); 40.74 (CH₂); 38,49 (CH).

### Beispiel 1c: Herstellung der Methoxysubstituierten Verbindungen

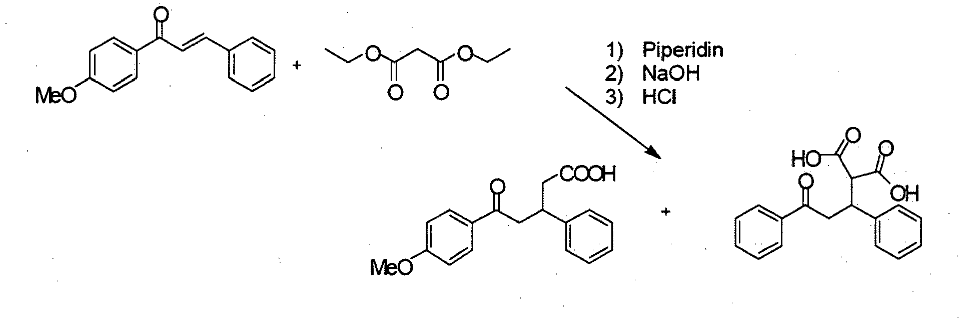

In einem Rundkolben mit Rückflusskühler wurde eine Lösung aus Methoxychalkon, Diethylmalonat und Piperidin in Ethanol erhitzt und unter Rückfluss 2 Tage lang gerührt.
Nach Abkühlen der Reaktionsmischung wurde kalte 4 M Natronlauge zugetropft, erhitzt und unter Rückfluss 6 Stunden lang gerührt. Anschließend wurde die Mischung abgekühlt, bei Raumtemperatur kalte konz. Salzsäure bis pH 1 zugetropft und das Reaktionsgemisch dreimal mit Diethylether extrahiert. Die vereinigten organischen Phasen wurden mit ges. NaCl-Lösung gewaschen, danach über MgSO₄ getrocknet und unter Vakuum vom Lösemittel befreit.

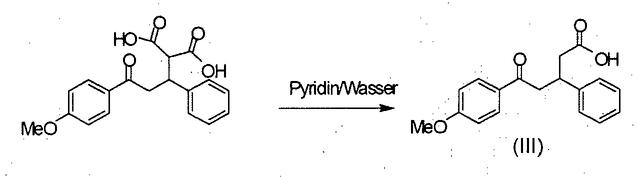

Da bei dieser Reaktion überwiegend die Dicarbonsäure entsteht, wurde das Produkt in einem Pyridin/Wasser-Gemisch (4:3) unter Rückfluss 2 Tage lang erhitzt. Nach der Aufarbeitung wurde ein ¹H-NMR-Spektrum aufgenommen in dem die charakteristischen Signale für das gewünschte Produkt nachzuweisen sind.
**¹H-NMR:** (300 MHz, C₂D₃N)
δ (ppm) = 7.95-7.90 (m, 10H); 3.80 (s, 3H); 3.50 (m, 1H); 3.43-3.41 (d, 2H); 2.75 (dd, 1H); 2.60 (dd, *J* = 15.9, 8.6Hz, 1 H).

### Beispiel 1d: Veresterungsreaktion zu Methyl-5-oxo-3,5-diphenylpentanoat (AB-02)

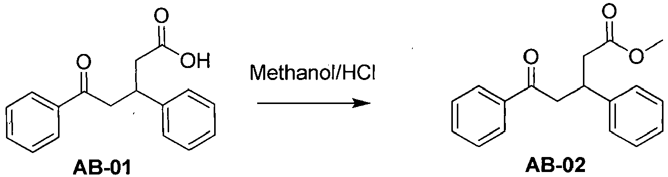

In einem 50 ml Rundkolben mit Rückflusskühler wurde eine Lösung aus der Carbonsäure in 7 ml Alkohol vorgelegt. Dazu wurde 0.1 ml HCl zugetropft und zum Rückfluss 6 h lang erhitzt.

Nach Abkühlen der Reaktionsmischung wurde 10 ml Eiswasser zugegeben und die Reaktionsmischung dreimal mit Ethylacetat extrahiert. Die vereinigten organischen Phasen wurden mit ges. Na₂CO₃-Lösung und mit Wasser gewaschen, danach über MgSO₄ getrocknet und vom Lösemittel befreit.
**M** = 282.33 g/mol
**GC/MS:** (50-300M), m/z: 282, 251, 209, 131, 105, 77, 51.
**t_{R}** (50-300M) = 10.751 min.
**¹H**-**NMR:** (300 MHz, C₂D₃N)
δ (ppm) = 7.94-7.18 (m, 10H); 3.79-3.72 (m, 1 H); 3.52 (d, *J* = 7.1 Hz, 2H); 3.41-2.65 (dd, 2H).
**¹³C**-**NMR:** (75 MHz, C₂D₃N):
δ (ppm) = 199.5 (C=0); 173.1 (C=O); 144.7 (Cq); 138.00 (Cq); 134.0-124.5 (6 Signale, C_{Ar}); 51.9 (CH₃); 45.0 (CH₂); 41.3 (CH₂); 38.6 (CH).

### Beispiel 1 e: Ethyl-5-oxo-3,5-diphenylpentanoat (AB-03)

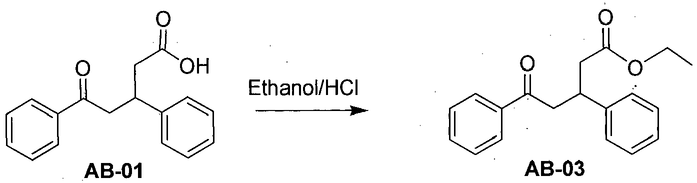

**M** = 296.36 g/mol
**GC/MS:** (50-300M), m/z: 296, 251, 109, 131, 105, 91, 77, 51.
**t_{R}** (50-300M) = 10.968 min
**¹H-NMR:** (300 MHz, C₂D₃N) δ (ppm) = 8.01-7.11 (m, 10H); 4.04-3.93 (q, *J* = 7.1 Hz, 2H); 3.85-3.71 (m, 1H); 3.39 (d, *J* = 7.1 Hz, 2H); 2.77-2.61 (dd, 2H); 1.09 (t, *J* = 7.1 Hz, 3H).
**¹³C-NMR:** (75 MHz, C₂D₃N):
δ (ppm) = 199.4 (C=O); 172.6 (C=O); 144.69 (Cq); 138.0 (Cq); 134.0 (C_{Ar}); 127.5 (6 Signale, C_{Ar}); 61.0 (CH₂); 45.1 (CH₂); 41.6 (CH₂); 38.7 (CH); 14.4 (CH₃).

### Beispiel 2: Belichtungsexperimente

### Beispiel 2a: Belichtungsreaktionen von 5-Oxo-3,5-diphenylpentansäure (AB-01)

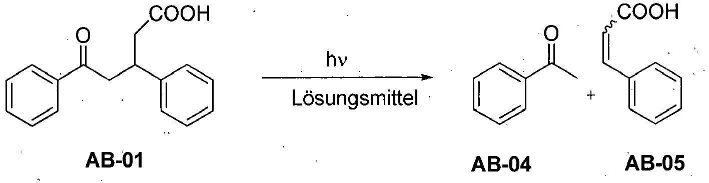

Nach AAV3 wurden 5.40 mg der 5-Oxo-3,5-diphenylpentansäure in jeweils 2 ml Methanol, Aceton und Acetonitril gelöst. Es wurde bei den Wellenlängen λ = 350 nm und 300 nm 5 Tage lang belichtet. Es wurden die Freisetzungsprodukte **AB**-**04** und **AB-05** erhalten. 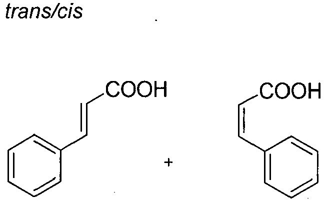 **¹H-NMR:** (300 MHz, C₂D₃N):
δ (ppm) = 7.60-7.57 (m, 2H); 7.45-7.40 (m, 3H);
*trans*: 7.80 (d, 1 H); 6.54-6.50 (d, 1 H); *cis:* 6.99 (d, 1 H); 5.96 (d, 1 H).

### Beispiel 2b: Belichtungsreaktionen von Methyl-5-oxo-3,5-diphenylpentanoat (AB-02)

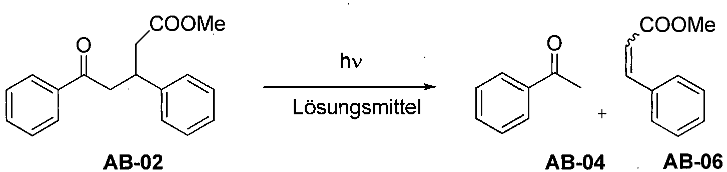

Es wurden 5.64 mg der 5-Oxo-3,5-diphenylpentansäure in jeweils 2 ml Methanol, Aceton und Acetonitril gelöst und bei den Wellenlängen λ = 350 nm und 300 nm 5 Tage lang belichtet.
Es wurden die Freisetzungsprodukte **AB-04** und **AB-06** erhalten. 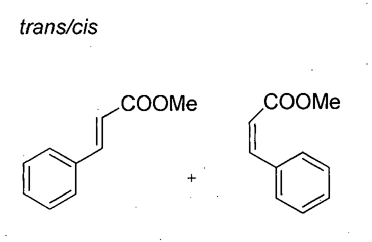 **¹H-NMR:** (300 MHz, CDCl₃)
δ (ppm) = 7.50 (m, 2H); 7.25 (m, 3H); 3.50 (s, 3H);
*trans*: 7.90 (d, 1 H); 6.50 (d, 1 H); *cis:* 7.00 (d, 1 H); 6.00 (d, 1 H).

### Beispiel 2c: Belichtung von Ethyl-5-oxo-3,5-diphenylpentanoat

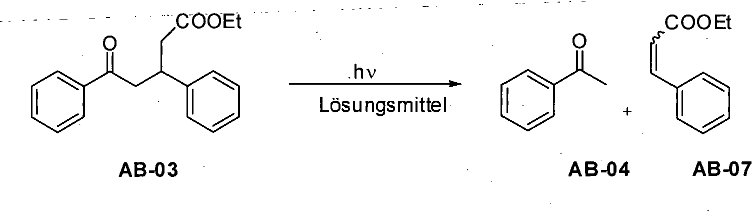

Es wurden 5.72 mg der 5-Oxo-3,5-diphenylpentansäure in jeweils 2 ml Methanol, Aceton und Acetonitril gelöst. Es wurde bei den Wellenlängen λ = 350 nm und 300 nm 5 Tage lang belichtet.
Es wurden die Freisetzungsprodukte **AB-04** und **AB-07** erhalten.

Die auf diese Weise hergestellten Verbindungen zeigten bei der Anwendung in Waschmitteln und Weichspülern bei der Textilbehandlung eine sehr gute Duftwirkung. Insbesondere wurde eine bessere Dauerhaftigkeit des Dufteindruckes auf der damit gewaschenen und danach getrockneten Wäsche gefunden, verglichen mit Waschmitteln und Weichspülern die eine äquimolare Menge der entsprechenden Zimtsäureester enthielten, ansonsten aber gleichgestaltet waren. Der frische Dufteindruck der Textilien hielt deutlich länger vor, sowohl nach Leinentrocknung wie insbesondere nach Trocknung im Maschinentrockner.

### UV-Vis Absorptionsspektren von am Chromophor substituierten 5-oxo-3,5-diphenylpentansäuren im Vergleich zu unsubstituierten

Die UV-Vis Spektren wurden mit einem Perkin Elmer Lambda 35 UV/Vis Spektrometer aufgenommen. Die Proben wurden dazu in Methanol gelöst (c = 0,1 mM) und in Quarz-Küvetten, die eine Schichtdicke von 1 cm besitzen, gemessen. Der gemessene Wellenlängenbereich betrug dabei 240 bis 440 nm. λₘₐₓ ist die Wellenlänge des Absorptionsmaximums der intensivsten Bande des jeweiligen Spektrums. 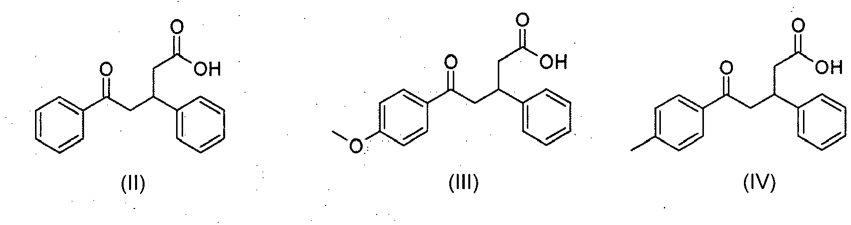 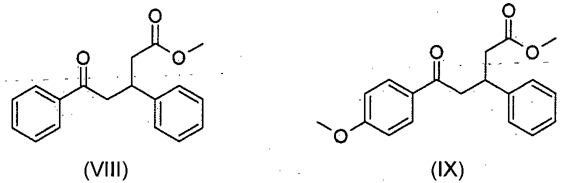 Die λₘₐₓ-Werte betragen:
λₘₐₓ(II) = 242 nm
λₘₐₓ(III) = 273 nm
λₘₐₓ(IV) = 254 nm
λₘₐₓ(VIII) = 243 nm
λₘₐₓ(IX) = 275 nm

## Patentansprüche

1. Verbindung der allgemeinen Formel (I), 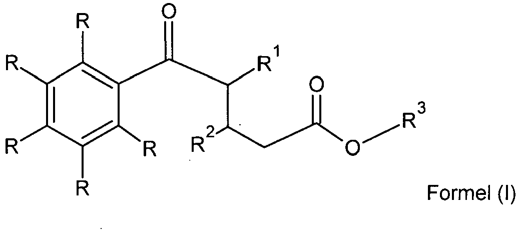 wobei
R1 für Wasserstoff, eine lineare oder verzweigte, substituierte oder unsubstituierte Alkyl-, Aryl-, oder Alkenylgruppe,
R2 und R3 unabhängig voneinander für eine lineare oder verzweigte, substituierte oder unsubstituierte Alkyl-, Aryl-, oder Alkenylgruppe, und
R jeweils, unabhängig voneinander, für Wasserstoff, eine Aminogruppe, -NO₂, eine lineare oder verzweigte, substituierte oder unsubstituierte Alkoxygruppe mit 1 bis 15 C-Atomen, eine lineare oder verzweigte, substituierte oder unsubstituierte Alkylgruppe mit 1 bis 15 C-Atomen, eine Cycloalkylgruppe, Acylgruppe, Arylgruppe, -OH, -NH₂, Halogen, NH-Alkyl oder -N(Alkyl)₂ stehen,
wobei der Substituent R3 eine n-Pentyl, sec-Pentyl-, Cinnamyl- oder Citronellylgruppe ist.

2. Verbindung nach Anspruch 1, **dadurch gekennzeichnet, dass** der Substituent R2 für eine substituierte oder unsubstituierte Arylgruppe, insbesondere für ein substituiertes oder unsubstituiertes Phenyl steht.

3. Verbindung nach Anspruch 1 oder 2, **dadurch gekennzeichnet, dass** der Substituent R1 für eine lineare oder verzweigte, substituierte oder unsubstituierte Alkylgruppe mit 1 bis 6 C-Atomen, vorzugsweise 1 bis 3 C-Atomen steht, insbesondere ein Methylrest ist.

4. Verbindung nach einem der Ansprüche 1 bis 4, **dadurch gekennzeichnet, dass** die Substituenten R jeweils unabhängig voneinander für Wasserstoff, Methyl- oder Methoxygruppen stehen.

5. Verbindung nach einem der Ansprüche 1 bis 4, entsprechend einer der folgenden Formeln (XX) bis (ILIV)

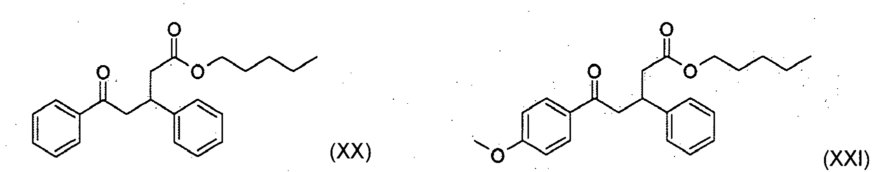

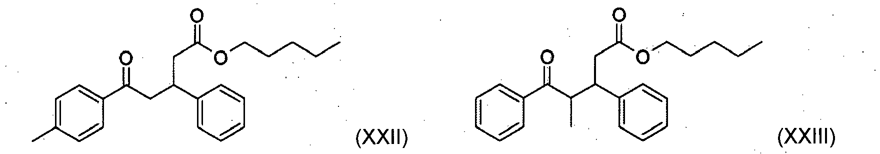

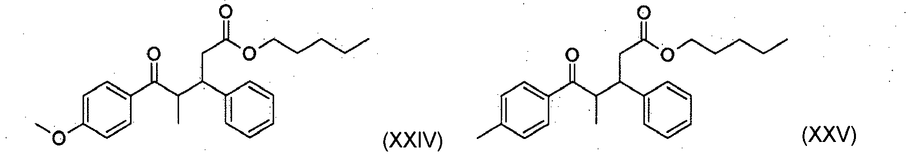

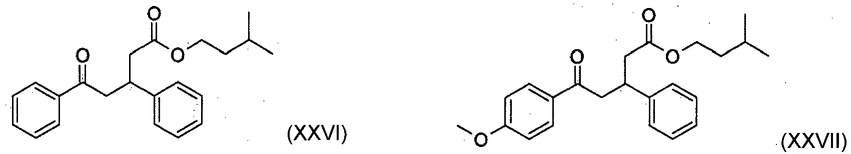

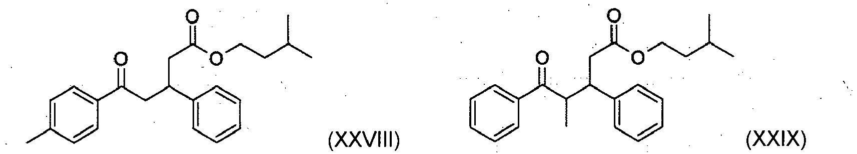

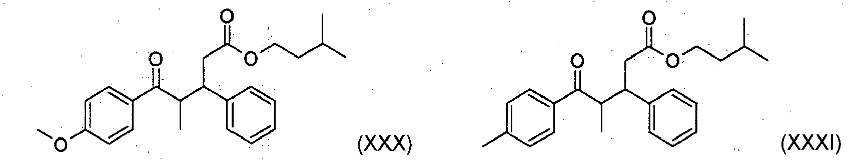

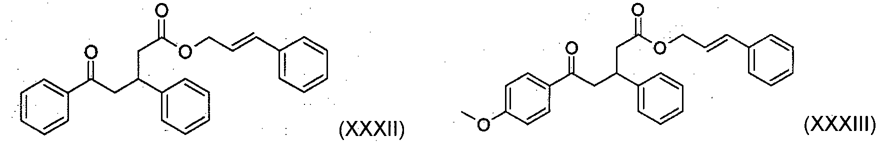

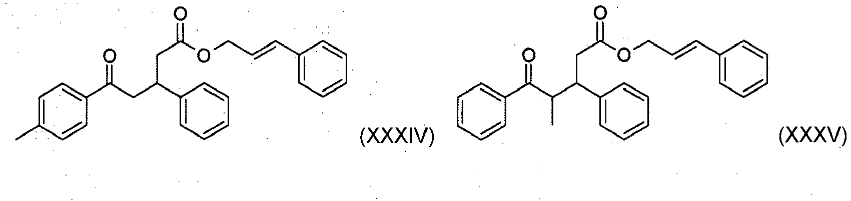

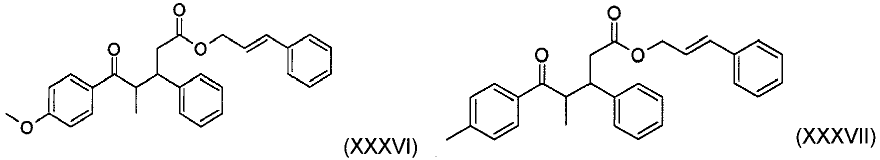

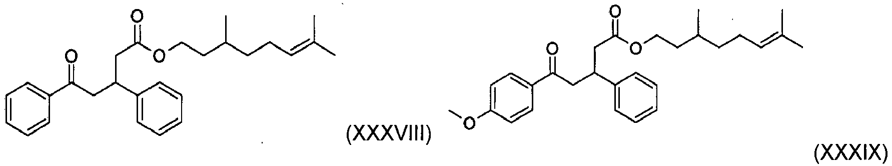

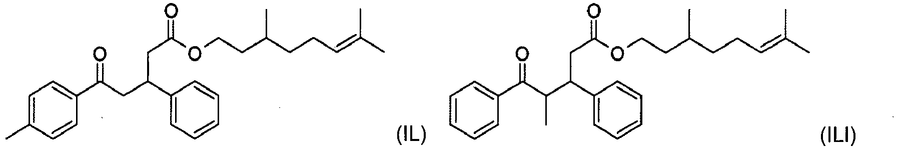

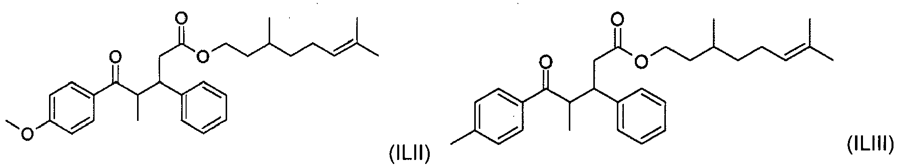

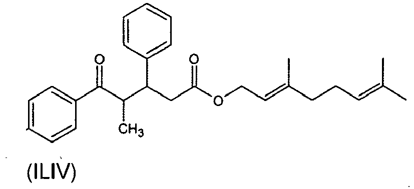

6. Wasch- oder Reinigungsmittel, enthaltend mindestens eine Verbindung nach einem der Ansprüche 1 bis 5.

7. Wasch- oder Reinigungsmittel nach Anspruch 6, wobei die genannte Verbindung in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,05 und 2 Gew.-%, jeweils bezogen auf das gesamte Mittel, enthalten ist.

8. Wasch- oder Reinigungsmittel nach einem der Ansprüche 6 oder 7, **dadurch gekennzeichnet, dass** es mindestens ein Tensid ausgewählt aus der Gruppe bestehend aus anionischen, kationischen, nichtionischen, zwitterionischen, amphoteren Tensiden und Mischungen daraus enthält.

9. Raumbeduftungsmittel, enthaltend mindestens eine Verbindung nach einem der Ansprüche 1 bis 5, wobei die genannte Verbindung vorzugsweise in Mengen zwischen 0,0001 und 50 Gew.-%, vorteilhafterweise zwischen 0,001 und 5 Gew.-%, weiter vorteilhaft zwischen 0,01 und 3 Gew.-%, insbesondere zwischen 0,05 und 2 Gew.-%, jeweils bezogen auf das gesamte Mittel, enthalten ist.

10. Kosmetisches Mittel, enthaltend mindestens ein Verbindung nach einem der Ansprüche 1 bis 5, wobei die genannte Verbindung vorzugsweise in Mengen zwischen 0,0001 und 50 Gew.-%, vorteilhafterweise zwischen 0,001 und 5 Gew.-%, weiter vorteilhaft zwischen 0,01 und 3 Gew.-%, insbesondere zwischen 0,05 und 2 Gew.-%, jeweils bezogen auf das gesamte Mittel, enthalten ist.

11. Verfahren zur lang anhaltenden Beduftung von Oberflächen, **dadurch gekennzeichnet, dass** eine Verbindung nach einem der Ansprüche 1 bis 5 oder ein Mittel nach einem der Ansprüche 6 bis 9 auf die zu beduftende Oberfläche aufgebracht wird und die genannte Oberfläche anschließend einer elektromagnetischen Strahlung umfassend die Wellenlängen von 200 bis 400 nm ausgesetzt wird.

## Claims

1. A compound of the general formula (I), 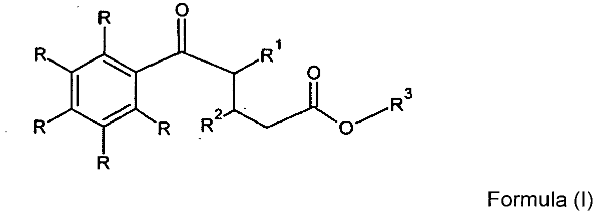 in which
R1 represents hydrogen, a linear or branched, substituted or unsubstituted alkyl group, aryl group or alkenyl group,
R2 and R3, independently of one another, represent a linear or branched, substituted or unsubstituted alkyl group, aryl group or alkenyl group, and
R in each case, independently of one another, represents hydrogen, an amino group, -NO₂, a linear or branched, substituted or unsubstituted alkoxy group having from 1 to 15 C atoms, a linear or branched, substituted or unsubstituted alkyl group having from 1 to 15 C atoms, a cycloalkyl group, acyl group, aryl group, -OH, -NH₂, halogen, NH alkyl or -N(alkyl)₂,
wherein the substituent R3 is an n-pentyl group, sec-pentyl group, cinnamyl group or citronellyl group.

2. The compound according to claim 1, **characterized in that** the substituent R2 represents a substituted or unsubstituted aryl group, in particular a substituted or unsubstituted phenyl.

3. The compound according to claim 1 or claim 2, **characterized in that** the substituent R1 represents a linear or branched, substituted or unsubstituted alkyl group having from 1 to 6 C atoms, preferably from 1 to 3 C atoms, in particular represents a methyl functional group.

4. The compound according to one of claims 1 to 4, **characterized in that** the substituent R, in each case independently of one another, represents hydrogen, methyl groups or methoxy groups.

5. The compound according to one of claims 1 to 4, corresponding to one of the following formulae (XX) to (ILIV)

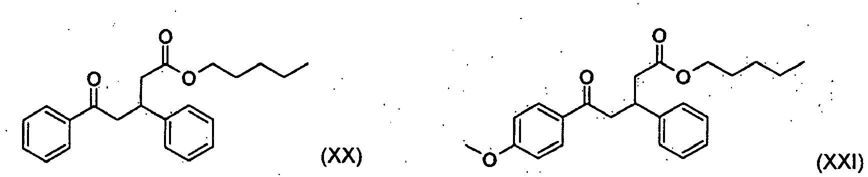

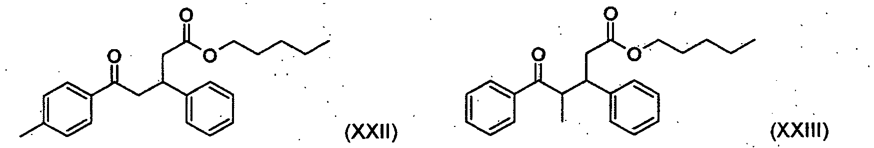

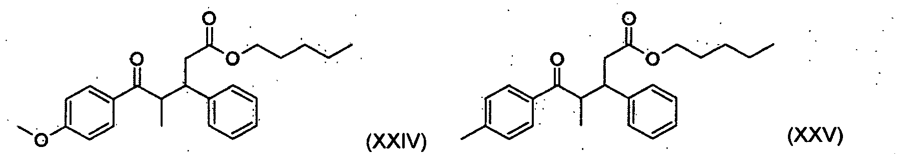

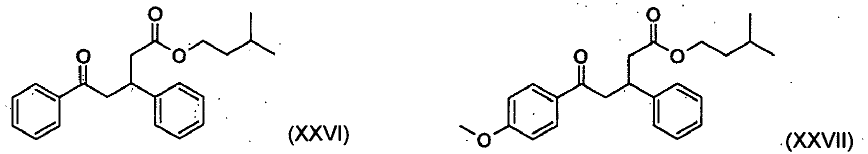

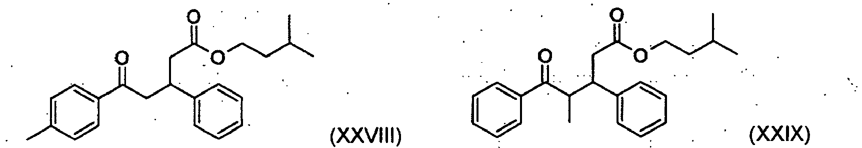

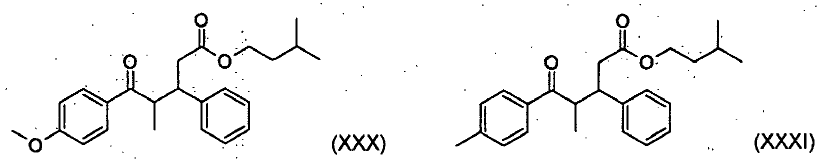

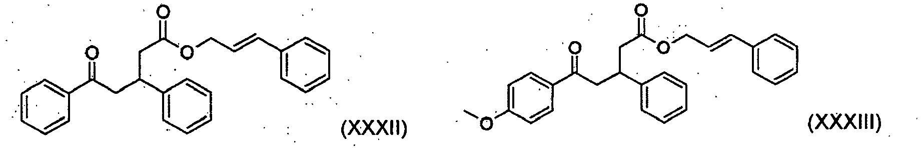

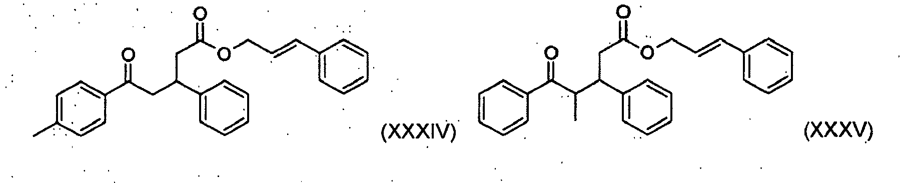

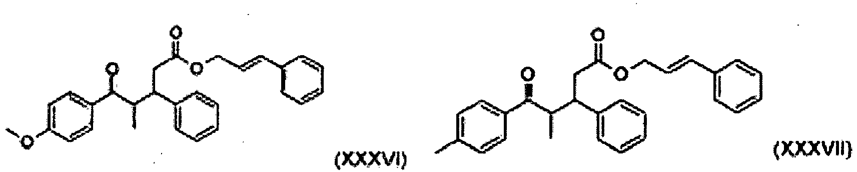

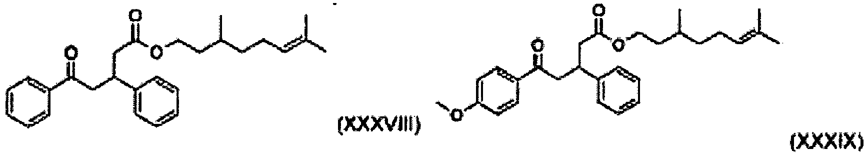

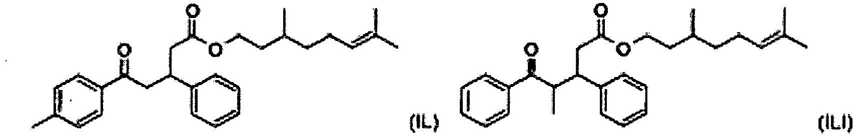

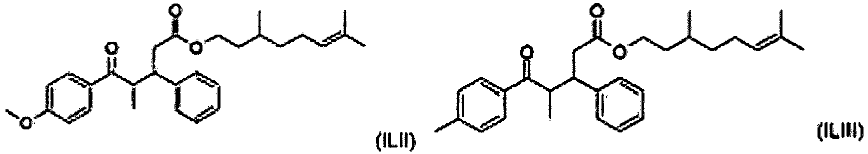

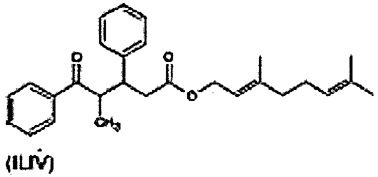

6. A detergent or cleaning agent containing at least one compound according to one of claims 1 to 5.

7. The detergent or cleaning agent according to claim 6, wherein said compound is contained in quantities of between 0.0001 and 5 wt.%, advantageously between 0.001 and 4 wt.%, more advantageously between 0.01 and 3 wt.%, in particular between 0.05 and 2 wt.%, in each case based on the total detergent or agent.

8. The detergent or cleaning agent according to one of claims 6 or 7, **characterized in that** it contains at least one surfactant selected from the group consisting of anionic, cationic, nonionic, zwitterionic, amphoteric surfactants and mixtures thereof.

9. A room-fragrancing agent, containing at least one compound according to one of claims 1 to 5, wherein said compound is preferably contained in quantities of between 0.0001 and 50 wt.%, advantageously between 0.001 and 5 wt.%, more advantageously between 0.01 and 3 wt.%, and in particular between 0.05 and 2 wt.%, in each case based on the total agent.

10. A cosmetic agent containing at least one compound according to one of claims 1 to 5, wherein said compound is preferably contained in quantities of between 0.0001 and 50 wt.%, advantageously between 0.001 and 5 wt.%, more advantageously between 0.01 and 3 wt.%, and in particular between 0.05 and 2 wt.%, in each case based on the total agent.

11. A method for fragrancing surfaces in a long-lasting manner, **characterized in that** a compound according to one of claims 1 to 5 or an agent according to one of claims 6 to 9 is applied to the surface to be fragranced and said surface is then exposed to electromagnetic radiation having wavelengths of from 200 to 400 nm.

## Revendications

1. Composé de formule générale (I), 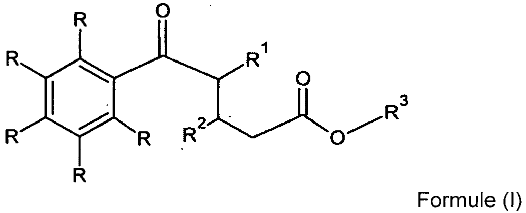
dans laquelle R1 représente l'hydrogène, un groupe alkyle, aryle ou alcényle linéaire ou ramifié, substitué ou non substitué,
R2 et R3 représentent indépendamment l'un de l'autre un groupe alkyle, aryle ou alcényle linéaire ou ramifié, substitué ou non substitué, et
R représente, dans chaque cas indépendamment, l'hydrogène, un groupe amine, -NO₂, un groupe alcoxy linéaire ou ramifié, substitué ou non substitué, comportant 1 à 15 atomes de carbone, un groupe alkyle linéaire ou ramifié, substitué ou non substitué comportant 1 à 15 atomes de carbone, un groupe cycloalkyle, un groupe acyle, un groupe aryle, -OH, -NH₂, un halogène, NH-alkyle ou -N(alkyle)₂,
le substituant R3 étant un groupe n-pentyle, sec-pentyle, cinnamyle ou citronellyle.

2. Composé selon la revendication 1, **caractérisé en ce que** le substituant R2 représente un groupe aryle substitué ou non substitué, en particulier un phényle substitué ou non substitué.

3. Composé selon la revendication 1 ou 2, **caractérisé en ce que** le substituant R1 représente un groupe alkyle linéaire ou ramifié, substitué ou non substitué comportant 1 à 6 atomes de carbone, de préférence 1 à 3 atomes de carbone, en particulier un radical méthyle.

4. Composé selon l'une quelconque des revendications 1 à 4 **caractérisé en ce que** les substituants R représente dans chaque cas indépendamment l'hydrogène, des groupes méthyle ou des groupes méthoxy.

5. Composé selon l'une quelconque des revendications 1 à 4 correspondant à l'une des formules (XX) à (ILIV) suivantes

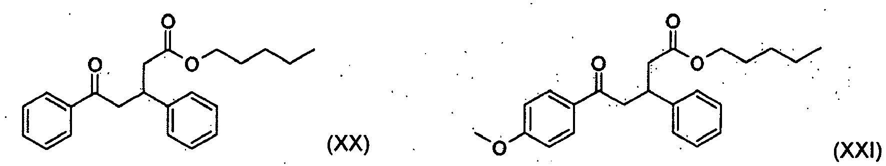

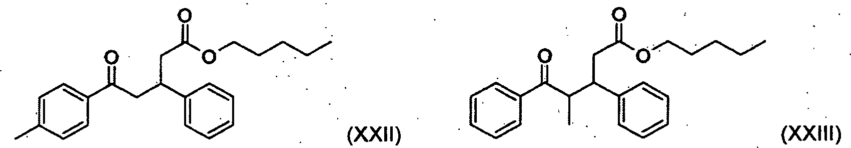

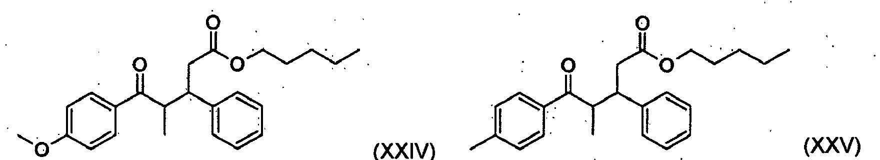

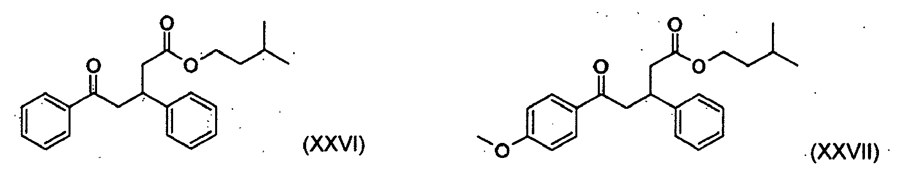

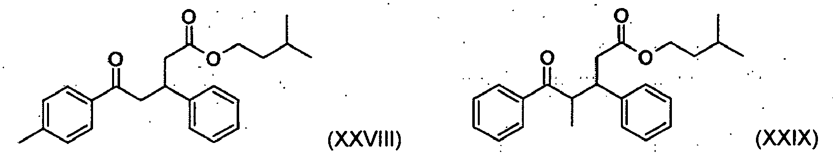

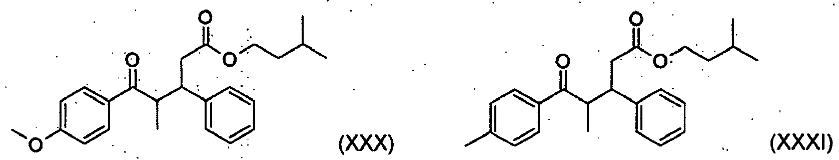

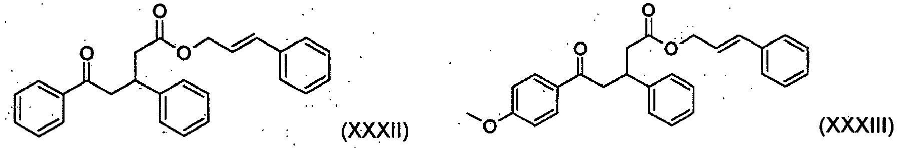

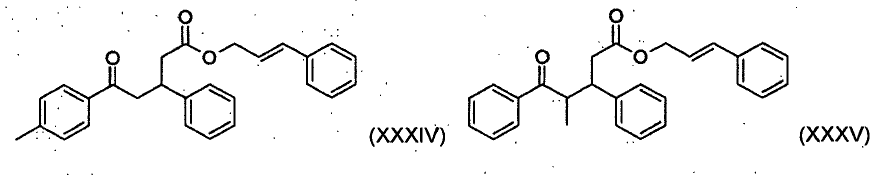

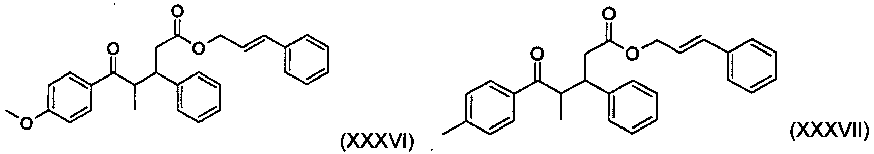

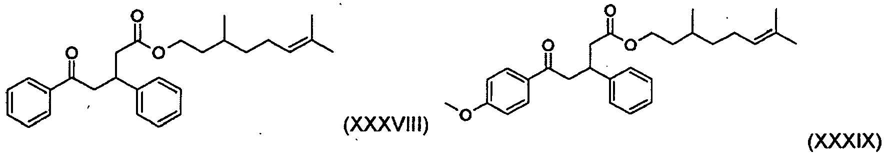

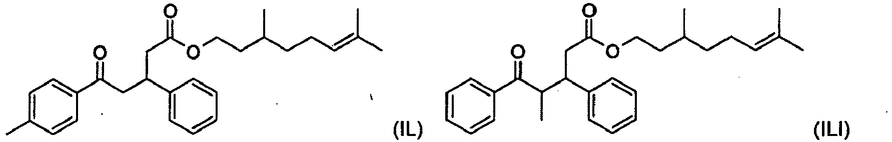

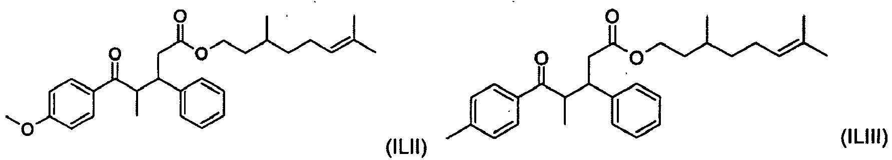

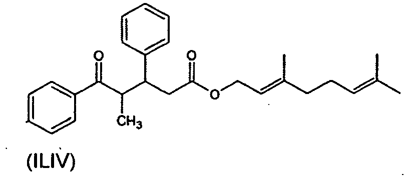

6. Agent détergent ou nettoyant comprenant au moins un composé selon l'une quelconque des revendications 1 à 5.

7. Agent détergent ou nettoyant selon la revendication 6, ledit composé étant présent dans des proportions comprises entre 0,0001 et 5 % en poids, avantageusement entre 0,001 et 4 % en poids, plus avantageusement entre 0,01 et 3 % en poids, en particulier entre 0,05 et 2 % en poids, par rapport au total de l'agent respectivement.

8. Agent détergent ou nettoyant selon la revendication 6 ou 7, **caractérisé en ce qu'**il contient au moins un tensio-actif choisi parmi le groupe composé de tensio-actifs anioniques, cationiques, non-ioniques, zwitterioniques, amphotère, et des mélanges de ceux-ci.

9. Agent de parfum d'ambiance comprenant au moins un composé selon l'une quelconque des revendications 1 à 5, ledit composé étant présent de préférence dans des proportions comprises entre 0,0001 et 50 % en poids, avantageusement entre 0,001 et 5 % en poids, plus avantageusement entre 0,01 et 3 % en poids et en particulier entre 0,05 et 2 % en poids, par rapport à la totalité de l'agent respectivement.

10. Agent cosmétique comprenant au moins un composé selon l'une quelconque des revendications 1 à 5, ledit composé étant présent de préférence dans des proportions comprises entre 0,0001 et 50 % en poids, avantageusement entre 0,001 et 5 % en poids, plus avantageusement entre 0,01 et 3 % en poids, en particulier entre 0,05 et 2 % en poids, par rapport à la totalité de l'agent respectivement.

11. Procédé pour parfumer des surfaces durablement, **caractérisé en ce qu'**un composé selon l'une quelconque des revendications 1 à 5 ou un agent selon l'une quelconque des revendications 6 à 9 est appliqué sur la surface à parfumer, et ladite surface est ensuite exposée à un rayonnement magnétique comprenant les longueurs d'ondes de 200 à 400nm.
